# EUROPÄISCHE PATENTSCHRIFT

(11) **EP 1 269 948 B1**
(45) Veröffentlichungstag und Bekanntmachung des Hinweises auf die Patenterteilung: **18.10.2006**
(21) Anmeldenummer: 02011168.8
(22) Anmeldetag: 18.05.2002
(51) Int. Cl.: A61F 13/15, A61F 13/551

(54) **Absorbierendes Hygieneprodukt sowie Verfahren und Vorrichtung zu seiner Herstellung**
Absorbent sanitary article, method and apparatus for making same
Article hygiénique absorbant, procédé et appareil pour sa production

(30) Priorität: 05.06.2001 DE 10127278
(43) Veröffentlichungstag der Anmeldung: 02.01.2003
(73) Patentinhaber: Winkler + Dünnebier Aktiengesellschaft, 56564 Neuwied (DE)
(72) Erfinder: Munsch, Klaus, 56566 Neuwied (DE)

(56) Entgegenhaltungen:
- EP-A- 0 472 376
- WO-A-99/23984
- US-A- 5 591 153
- US-B1- 6 234 229

## Beschreibung

Die Erfindung betrifft ein Hygieneprodukt sowie ein Verfahren und eine Vorrichtung zu seiner Herstellung.

Insbesondere bei der Herstellung von mehrlagigen, je einen Kern aus saugfähigem Werkstoff und beidseitig Decklagen aufweisenden Damenbinden ist es bekannt, zunächst Roh- bzw. Vorprodukte herzustellen, das heißt Produkte zunächst zu schaffen, zu deren Vervollständigung noch mindestens ein weiterer Verfahrensschritt erforderlich ist. Dazu werden die Vorprodukte mit Hilfe einer mindestens eine Silikon-Trennschicht aufweisenden Folie zusätzlich noch partiell mit Klebstoff versehen. Sodann werden die derart vervollständigten Hygieneprodukte einzeln in die Folie verpackt, die auch als Träger und zum Auftragen des Klebstoffes dient. Auch ist es bekannt, die Hygieneprodukte vor dem Verpacken zu falten und / oder zunächst die Folie gezielt durch Prägen zu fixieren, woraufhin die Prägestellen zum Vereinzeln der Hygieneprodukte durchtrennt werden.

Bei diesem Stand der Technik wird die Silikon-Trennschicht jeweils als sich quer erstreckender Streifen auf bahnförmige Kunststoff-Folie aufgebracht und auch der Klebstoff bzw. die Leimstreifen liegen quer auf der Folienbahn bzw. auf den Silikon-Streifen. Das Zusammenführen der Folie mit dem fast vollständig fertiggestellten Hygieneprodukt erfolgt dabei derart, daß das Vorprodukt der sich in ihrer Längsrichtung bewegenden Kunststoff-Folie querliegend zugeführt wird. Sobald der Klebstoff das Vorprodukt berührt und an diesem haftet, ist das Hygieneprodukt fertiggestellt, wobei es gleichzeitig in einem Faltkanal in die Folie eingehüllt und zugleich gefaltet wird. Die Faltlinien des Hygieneproduktes liegen dabei in Bewegungsrichtung der noch bahnförmigen Folie. Zugleich bildet die Folie einen sich kontinuierlich schließenden Schlauch. Jeweils zwischen zwei mit Klebstoff versehenen Hygieneprodukten wird der Schlauch sodann noch mit Prägelinien verschlossen und schließlich im Bereich der Prägelinien durchtrennt. Die Hygieneprodukte sind daraufhin einzeln verpackt.

Diese Art der Herstellung setzt nicht nur voraus, daß die fast fertiggestellten Vorprodukte aus einer Lage in Längsrichtung der Produktionslinie in eine Querlage überführt werden müssen, sondern es ist auch notwendig, daß der Klebstoff sehr genau auf die Folie aufgebracht wird und daß die Teile absolut exakt zueinander, das heißt registergenau zusammengeführt werden. Andernfalls ließe sich der Klebstoff nicht exakt auf die vorbestimmten Stellen auf den Vorprodukten übertragen. Wechselnde Geschwindigkeiten und Abstände der Vorprodukte bei ihrer Bewegung in Längsrichtung und dann in Querrichtung sowie das Zusammenwirken mit der zugeführten und bereits partiell mit Klebstoff versehenen Folie erschweren die Fertigstellung erheblich. Alternativ wäre es nur möglich, die Folie ganzflächig mit Silikon zu versehen. Dies würde aber dann dazu führen, daß das Verprägen der Folienränder schwierig wäre.

Der Erfindung liegt daher die Aufgabe zugrunde, Maßnahmen vorzusehen, um das Herstellungsverfahren und insbesondere das Aufbringen des Klebstoffes auf eine der Decklagen des Kerns sowie das Verpacken einzelner Produkte entscheidend zu vereinfachen. Daraus sollen sich ferner auch Vereinfachungen der zur Herstellung der Produkte dienenden Vorrichtung und schließlich zweckmäßige Verbesserungen des Produktes selbst ergeben.

Zur Lösung dieser Aufgabe sieht die Erfindung vor, daß zunächst Klebstoff auf eine mit einer Silikon-Trennschicht versehene, bahnförmige Folie in Längsrichtung sowie partiell aufgebracht wird und daß die Folie und die Vorprodukte sodann ebenfalls in Längsrichtung einander zugeführt und aufeinandergelegt werden und daß die dabei fertiggestellten Hygieneprodukte schließlich bei weiterer Bewegung in Längsrichtung in die Folie verpackt und / oder zuvor gefaltet werden

Der Produktionsfluß ist aufgrund der besagten Verfahrensschritte deutlich einfacher und weniger aufwendig als bisher. Vor allem aber wirkt sich eine ungenaue Lage der Teile zueinander beim Aufeinandertreffen weniger störend aus. Dies gilt insbesondere dann, wenn eine gezielt veranlaßte, versetzte Anordnung von Folie und Produkt zueinander bei einer erfindungsgemäßen Verwendung dieses Verfahrensschrittes zu einer neuen Produktlonsmöglichkeit und zu einem höherwertigen Produkt führt als bisher.

Auch die Produktionsgeschwindigkelt läßt sich gegenüber der bisherigen Arbeitsweise steigern, so daß eine einheitliche Produktion in Längsrichtung der Teile gegenüber einer Produktion in Längsrichtung und in Querrichtung wie bisher vorteilhaft ist.

Bei einem aus der EP 0472376 A1 bekannten Hygieneprodukt der hier interessierenden Art wie beispielsweise einer Damenbinde sind beidseitig Decklagen auf einem saugfähigen Kern angeordnet und bilden ein Vorprodukt, das auch eine Silikon- Trennschicht und Klebstoff aufweist und in einer Folie angeordnet ist, die als Verpackung für das in Lagen gelegte, einzeln verpackte Produkt vorgesehen ist.

Um das besonders zweckmäßige Herstellungsverfahren gemäß der Erfindung durchführen zu können, ist für das Hygieneprodukt vorgesehen, dass die Silikon- Trennschicht in Längsrichtung auf der zugleich als Verpackung dienenden Folie angeordnet ist und dass sich der Klebstoff auf der Silikon-Trennschicht befindet.

Der Grundgedanke der Erfindung besteht bezüglich des Produktes somit darin, dass sowohl die Silikon-Trennschicht als auch die Klebstoff-Schicht auf der als Verpackung dienenden Folie in Längsrichtung des Produktes und aufeinander angeordnet sind. Dies ist beim Gegenstand der EP 0472376 A1 nicht der Fall. Bei den beiden dort beschriebenen und dargestellten Varianten sind die Klebstoff-Flächen und die daneben sowie parallel angeordneten Silikon-Trennflächen auf einer Decklage vorgesehen, die den beidseitig vorgesehenen Decklagen beim erfindungsgemäßen Produkt entsprechen. Diese Decklagen umgreifen ferner die Kernschicht mit ihren jeweils verprägten Rändern.

Weitere Einzelheiten und Merkmale der Erfindung gehen aus Unteransprüchen und zwecks Erläuterung aus der Beschreibung und der Zeichnung hervor.

Die Erfindung wird nachstehend anhand von Ausführungsbeispielen, die in der Zeichnung dargestellt sind, näher beschrieben. Dabei zeigen:
- Fig. 1: in Seitenansicht eine Prinzipskizze einer zum Stand der Technik gehörenden Vorrichtung;
- Fig. 2: eine Draufsicht auf die Vorrichtung gemäß Figur 1;
- Fig. 3: eine Draufsicht auf ein Hygieneprodukt/Damenbinde mit einem zugehörigen Folienstück gemäß einer bevorzugten Ausführungsform der Erfindung;
- Fig. 4: schematisch eine Seitenansicht der Teile gemäß Fig. 3;
- Fig. 5: schematisch in Seitenansicht ein einzeln verpacktes Hygieneprodukt gemäß Fig. 3;
- Fig. 6: eine Draufsicht auf das einzeln verpackte Hygieneprodukt gemäß Fig. 3;
- Fig. 7: in Seitenansicht eine schematische Darstellung der Vorrichtung zum Auftragen von Klebstoff auf ein Vorprodukt und zum Verpacken eines einzeln verpackten Endproduktes, das heißt des vollständig fertiggestellten Hygieneproduktes in eine Folie gemäß einem ersten Ausführungsbeispiel;
- Fig. 8: die Verfahrensschritte der Vorrichtung nach Fig. 7 jeweils schematisch in Seitenansicht und in Drauf sicht;
- Fig. 9: eine Ansicht wie in Fig. 7 von einer abgewandelten Ausführungsform und
- Fig. 10: die Verfahrensschritte der Vorrichtung gemäß Fig. 9 jeweils schematisch in Seitenansicht und in Draufsicht;
- Fig. 11: eine Ansicht wie in den Fig. 7 und 9 von einem wei-teren Ausführungsbeispiel;
- Fig. 12: jeweils nur zum Teil in Seitenansicht und in Draufsicht sowie schematisch die Verfahrensschritte der Vorrichtung gemäß Fig. 11 und
- Fig. 13: eine Ansicht wie in den Fig. 7, 9 und 11 von einem letzten Ausführungsbeispiel der Vorrichtung.

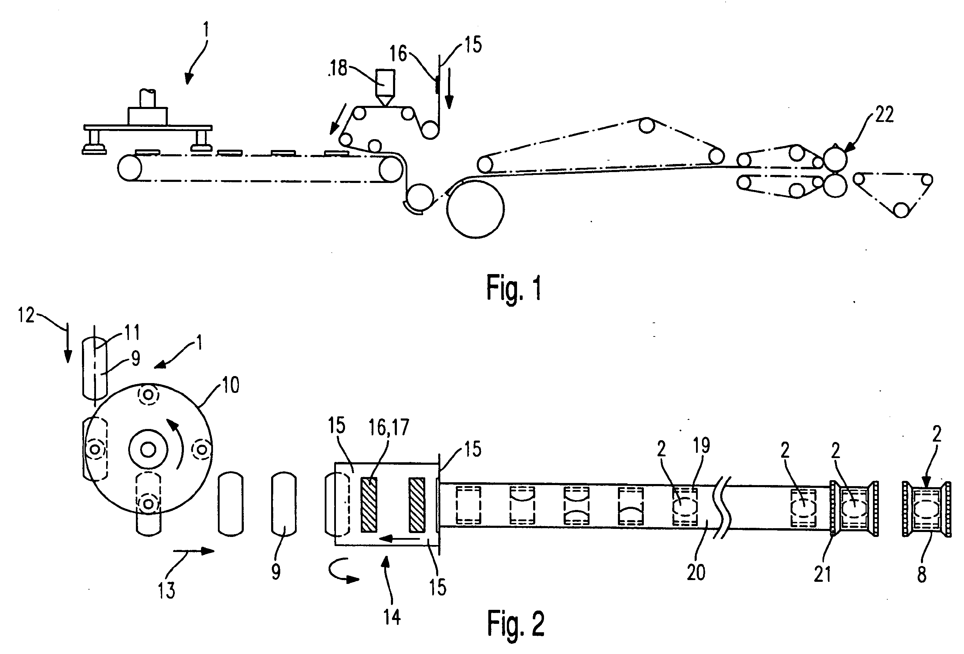

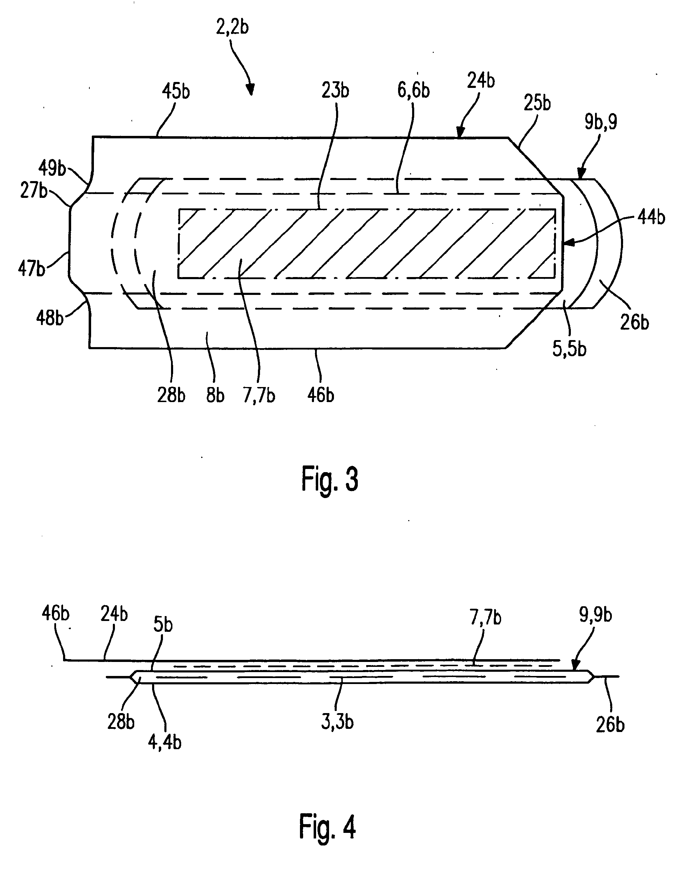

Eine bekannte Vorrichtung 1 zum Herstellen von Hygieneprodukten 2 (zum Beispiel von Binden), die - wie im Falle der Darstellung in den Fig. 3 und 4 - je einen Kern 3 und beidseitig Decklagen 4 und 5 sowie auf einer der Decklage 5 eine Klebstoffschicht 7 aufweisen und in eine Folie 8 einzeln verpackt werden, umfaßt eine in den Fig. 1 und 2 nicht dargestellte Einrichtung, die den mit den beiden Decklagen 4 und 5 versehenen Kern 3 als Vorprodukt 9 herstellt und gemäß den Fig. 1 und 2 einem Drehteller 10 zuführt. Die mit ihrer Längsrichtung bzw. Längsachse 11 in Transportrichtung gemäß dem Pfeil 12 vom Drehteller 10 übernommenen Vorprodukte 9 verlassen den Drehteller 10 nach einer Vierteldrehung und werden dann quer liegend gemäß dem Stand der Technik entsprechend dem Pfeil 13 einer Station 14 zugeführt. In der Station 14 wird eine Folie in Gestalt einer Folienbahn 15 auf die Vorprodukte 9 aufgelegt. Die Folienbahn 15 weist in partieller Anordnung quer liegende Silikon-Streifen 16 als Silikon-Trennschicht 6 auf und trägt auf den Silikon-Streifen 16 den sich jeweils ebenfalls quer zur Längsrichtung der Folienbahn 15 erstreckenden Klebstoff 7 in Form von Klebstoffstreifen 17. Diese Klebstoffstreifen 17 müssen von einer Klebstoff - Auftrageeinrichtung 18 taktgenau auf die Silikon-Streifen 16 aufgetragen werden. Nachdem die Folienbahn 15 in der Station 14 auf die hier quer zugeführten Vorprodukte 9 aufgelegt ist, werden die Vorprodukte 9 zusammen mit der aufliegenden Folienbahn 15 zu einem mindestens zweilagigen, vorzugsweise dreilagigen Päckchen 19 gefaltet und gleichzeitig in die Folienbahn 15 eingehüllt. Dabei bildet die Folienbahn 15 einen das Hygieneprodukt 2 umgebenden Schlauch 20. In Transportrichtung vor und hinter dem Hygieneprodukt 2 wird der Schlauch 20 sodann noch verprägt und dadurch vor und hinter dem Hygieneprodukt 2 verschlossen. Im Bereich der Prägestellen 21 wird der Schlauch 20 sodann mit Hilfe einer Trennvorrichtung 22 durchtrennt, woraufhin das Hygieneprodukt 2 gemäß dem Stand der Technik einzeln verpackt ist (Fig.2).

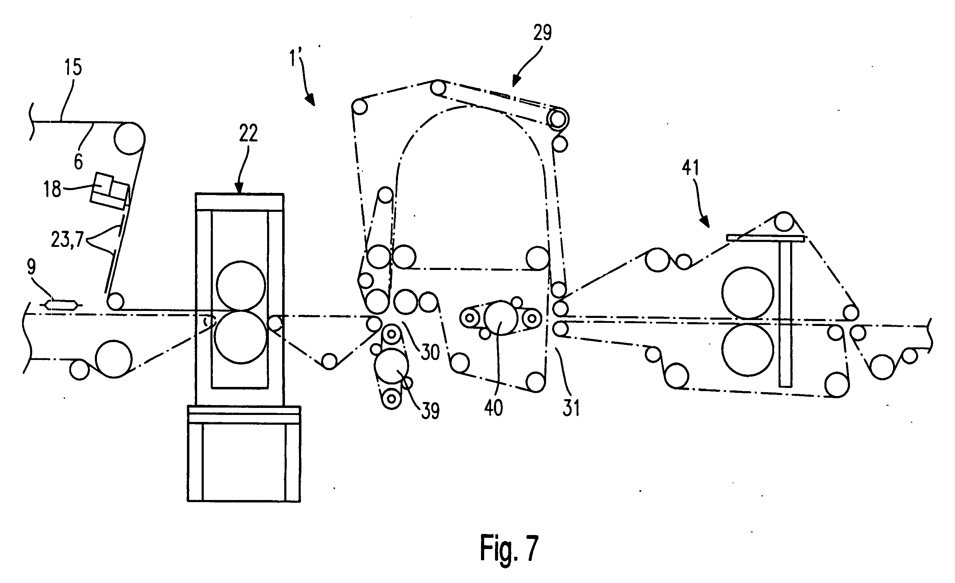

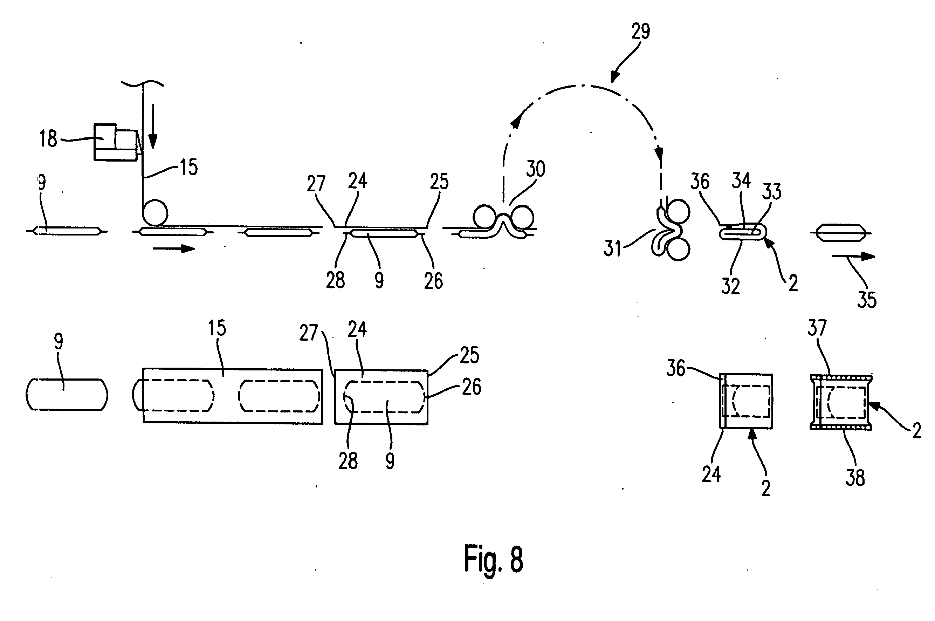

Eine erfindungsgemäße Vorrichtung 1' (Fig. 7) zum Herstellen von Hygieneprodukten 2 aus Vorprodukten 9 umfaßt zum Auftragen von Klebstoff 7 auf das Vorprodukt 9 eine Klebstoff-Auftrageeinrichtung 18, die ebenfalls zunächst den Klebstoff 7 auf eine Silikon-Trennschicht 6 auf der Folienbahn 15 partiell aufträgt. Die Silikon-Trennschicht 6 befindet sich als schmaler Längsstreifen auf der Folienbahn und kann bereits vom Hersteller der Folienbahn auf diese aufgebracht werden. Ebenso wie der Silikon-Streifen sich in Längsrichtung der Folienbahn 15 erstreckt, gilt dies auch für den von der Klebstoff-Auftrageeinrichtung 18 aufgebrachten Klebstoff 7, der jedoch nicht als durchgehender Streifen auf die Folienbahn 15 aufgetragen wird, sondern jeweils als Streifenstücke 23, die kürzer sind als das Vorprodukte 9 bzw. als das fertiggestellte Hygiene-Vorprodukt 2 (Fig. 3 und 8).

Die mit Klebstoff 7 versehene Folienbahn 15 wird sodann gemäß Fig. 7 auf das bzw. die Vorprodukte 9 aufgelegt und zusammen mit diesen einer Trennvorrichtung 22 zugeführt. Dort wird die Folienbahn 15 durchtrennt, so daß sich jeweils Folienstücke 24 auf den Vorprodukten 9 befinden (Fig. 8). Die Trennschnitte können dabei derart ausgeführt werden, daß das in Transportrichtung vordere Folienstückende 25 wenig über das vordere Produktende 26 vorsteht, während das hintere Folienstückende 27 deutlich über das hintere Produktende 28 übersteht (Fig. 8). In dieser Lage zueinander gelangt das Vorprodukt 9 mit dem Folienstück 24 in eine Faltvorrichtung 29, die sowohl in Fig. 7 als auch in Fig. 8 nur schematisch dargestellt ist. Gemäß dem Ausführungsbeispiel wird das Vorprodukt 9 zusammen mit dem Folienstück 24 an zwei Stellen 30 und 31, das heißt zweimal gefaltet, so daß es die Faltvorrichtung 29 mit drei aufeinander liegenden Lagen 32, 33, und 34 verläßt. Außerdem ist das Hygieneprodukt 2 zugleich in das Folienstück 24 eingehüllt, dessen in Transport- bzw. Laufrichtung gemäß Pfeil 35 hinteres Folienstückende 27 zum Beispiel als Öffnungslasche etwas über das Ende der obersten Lage 34 vorsteht. In diesem Verfahrenstadium ist das zugleich zum Verpacken dienende Folienstück 24 nur noch nicht gegen Öffnen gesichert, wozu das Folienstück 24 schließlich noch im Bereich der in Transportrichtung 35 außen liegenden Seitenrädern 37 und 38 verprägt wird. Damit ist das Hygieneprodukt 2 einzeln sowie sicher in dem Folienstück 24 fixiert bzw. verpackt.

Die Faltvorrichtung 29 umfaßt zur Herstellung eines dreilagigen Produktes zwei Stößeleinrichtungen 39 und 40, wie aus Fig. 7 hervorgeht. Ferner sind mehrere Transporteinrichtungen und Führungsrollen vorgesehen, auf die es hier im einzelnen nicht ankommt. Grundsätzlich dasselbe gilt für die Vorrichtung 41, in der die Seitenränder 37 und 38 des gefalteten und in das Folienstück 24 eingepackten Produktes durch Prägen verschlossen werden. Sowohl die Faltvorrichtung 29 als auch die Vorrichtung 41 zum Prägen gehören zum Stand der Technik und sind nicht Gegenstand der Erfindung.

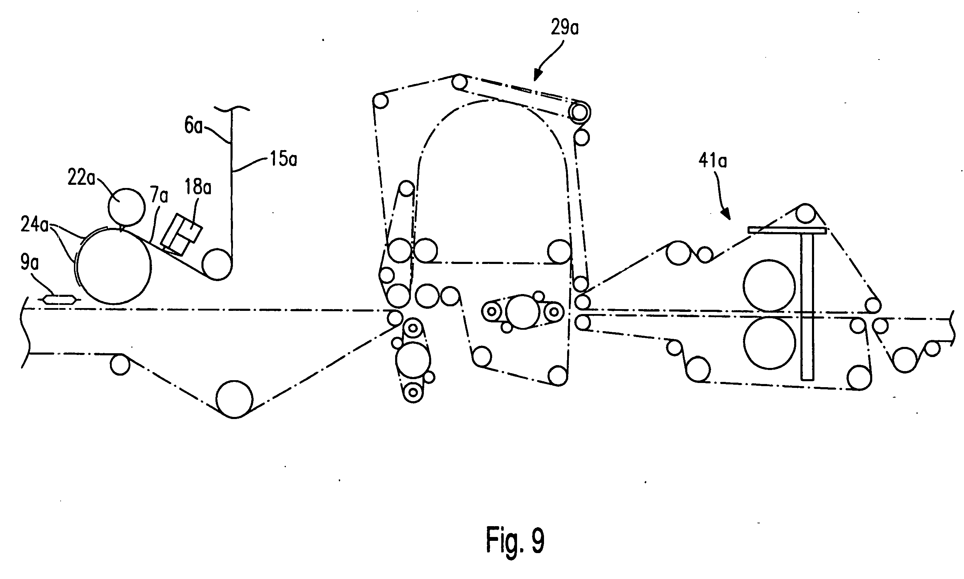

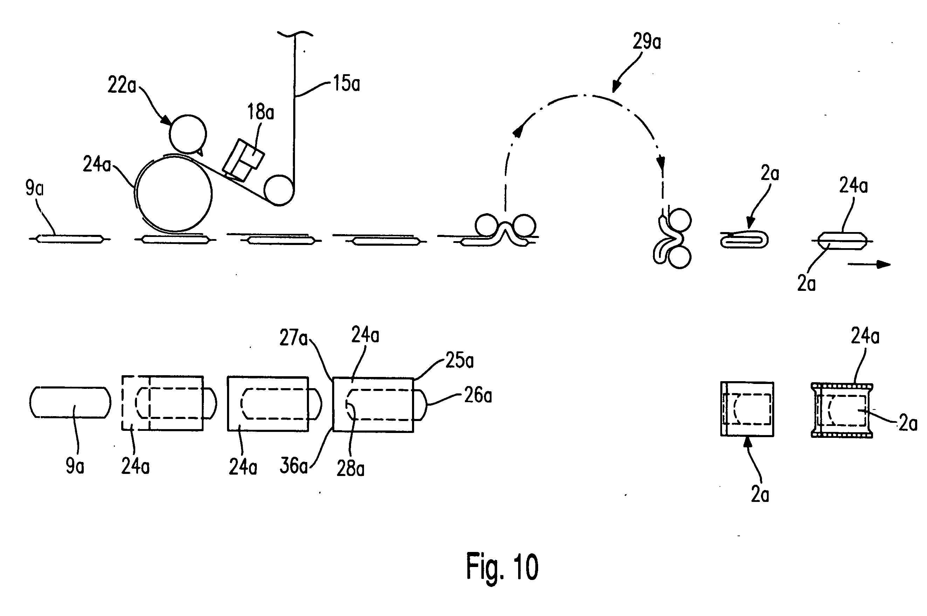

Das Durchtrennen der Folienbahn 15 muß nicht zwingend erst dann erfolgen, nachdem die Folienbahn 15 auf die Vorprodukte 9 aufgelegt wurde. Gemäß einer Abwandlung des erfindungsgemäßen Verfahrens ist es auch möglich, ein bereits vereinzeltes Folienstück 24 a auf ein Vorprodukt 9 a aufzulegen, wie dies aus Fig. 9 in Verbindung mit Fig. 10 hervorgeht.

Für dieses und für alle folgenden Ausführungsbeispiele gilt, daß grundsätzlich gleichartige Teile jeweils dieselben Bezugszahlen und zusätzlich einen Buchstabenindex aufweisen.

Gemäß dem in den Fig. 9 und 10 dargestellten Ausführungsbeispiel wird die Folienbahn 15a bereits unmittelbar hinter der Klebstoff-Auftrageeinrichtung 18a mit Hilfe einer Trennvorrichtung 22a in einzelne Folienstücke 24 a unterteilt. Sodann wird das mit einer Silikon-Trennschicht 6a und Klebstoff 7a versehene Folienstück 24a auf das Vorprodukt 9a aufgelegt, und die Teile werden dann gemeinsam wie bei dem zuerst beschriebenen Verfahren der Faltvorrichtung 29a und der Vorrichtung 41a zum Prägen bzw. zum Verschließen der nunmehr als Verpackung dienenden Folienstücke 24a zugeführt.

Einen Vorteil bietet das Verfahren, gemäß dem bereits vereinzelte Folienstücke 24a auf die Vorprodukte 9a aufgelegt werden, vor allem insofern, weil es möglich ist, die Folienstücke 24a versetzt auf die Vorprodukte 9a aufzulegen (siehe Draufsicht in Fig. 10). Zweckmäßigerweise geschieht dies gemäß dem in Fig. 10 dargestellten Produktbeispiel derart, daß das vordere Produktende 26a deutlich über das vordere Folienstückende 25a vorsteht, während das hintere Folienstükkende 27a über das hintere Produktende 28a vorsteht. Das hintere Folienstückende 24a kann daher wiederum als Aufreißlasche dienen, wie dies auch bei dem oben beschriebenen Ausführungsbeispiel der Fall ist.

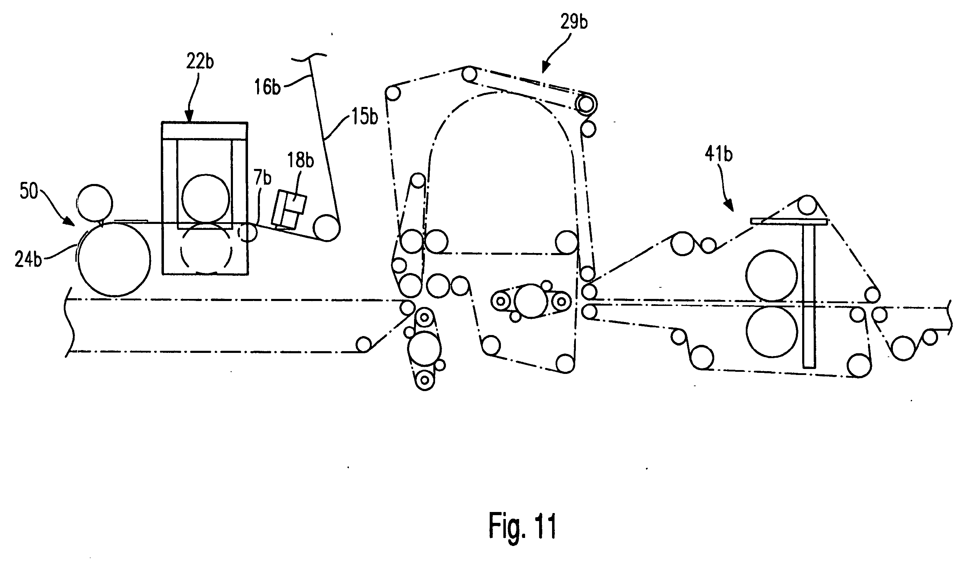

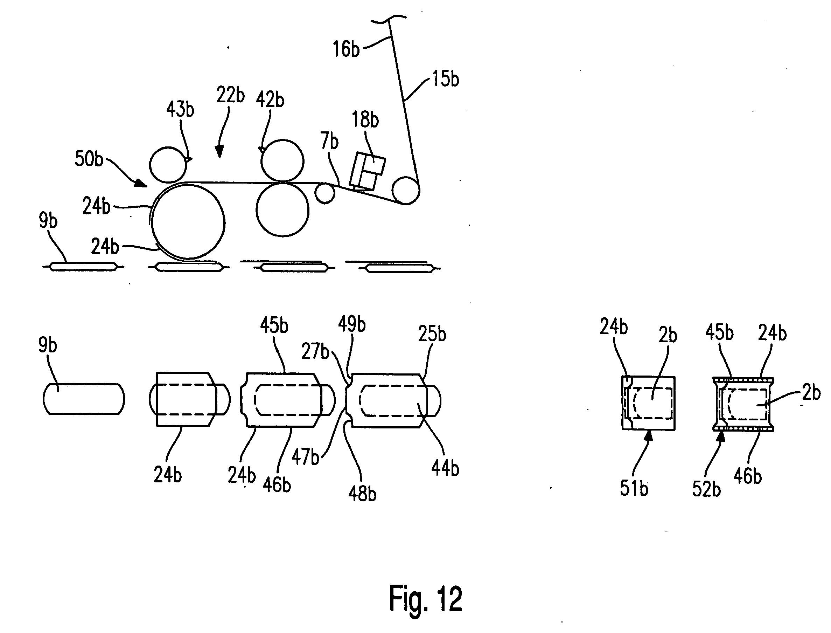

Gemäß einem weiteren, in den Fig. 11 und 12 dargestellten Ausführungsbeispiel wird ebenfalls die mit einem Silikonstreifen 16b versehene Folienbahn 15b zweckmäßigerweise nach dem Auftragen von Klebstoff 7b mit Hilfe einer Klebstoff-Auftrageeinrichtung 18b in einzelne Folienstücke 24b unterteilt. Dazu dient eine Trennvorrichtung 22b, die jedoch nicht nur gerade Schnitte quer zur Laufrichtung der Folienbahn 15b erzeugt, wie im Falle des in den Fig. 9 und 10 dargestellten Ausführungsbeispieles, sondern Formschnitte beziehungsweise Konturschnitte bzw. Konturschnitte und Trennschnitte an beziehungsweise für die beiden Enden der Folienstücke 24b herstellt. Dazu weist die Trennvorrichtung 22b gemäß dem in den Fig. 11 und 12 ebenfalls nur schematisch dargestellten Ausführungsbeispiel zwei Messer 42b und 43b auf, die im Abstand voneinander wirksam sind.

Die Form und Gestalt der Messer 42b und 43 sind vorzugsweise derart unterschiedlich, daß das in Laufrichtung vordere Messer 42b seitliche Konturschnitte in der Folienbahn 15b und das andere Messer 43b Trennschnitte zum Vereinzeln der Folienstücke 24b durchführen.

Gemäß dem in den Fig. 3 und 12 dargestellten Ausführungsbeispiel wird die Kontur an den beiden Folienstückenden 25b und 27b zweckmäßigerweise derart gewählt, daß der in Transportrichtung bzw. Längsrichtung mittige Bereich 44b des Folienstückes 24b an mindestens einem Ende über mindestens einen der seitlichen Ränder 45b beziehungsweise 46b in Laufrichtung vorsteht und / oder entgegen der Transportrichtung vorsteht. Die seitlichen Ränder 45b und 46b sind daher gemäß Ausführungsbeispiel gegenüber dem mittigen Bereich 44b zurückgenommen bzw. kürzer als der mittige Bereich 44b des Folienstückes 24b.

Das bei der Herstellung in Transportrichtung vordere Folienstückende 25b ist gemäß Ausführungsbeispiel trapezförmig. Das hintere Folienstückende 27b weist ein gerades, mittleres Randstück 47b und daneben jeweils bogenförmige Randstücke 48b und 49b auf, die in die seitlichen Ränder 45b und 46b auslaufen.

Nachdem das Folienstück 24b in der Station 50b auf das Vorprodukt 9b aufgelegt worden ist, haftet der Klebstoff 7b auf dem Vorprodukt 9b mit der Folge, daß das Hygieneprodukt 2 fertiggestellt ist. Das Hygieneprodukt 2 gelangt daraufhin in die Faltstation 29b und wird dort gemäß Ausführungsbeispiel in drei aufeinander liegende Lagen 32b, 33b und 34b gelegt und ist gleichzeitig in das Folienstück 24b eingehüllt. Das Hygieneprodukt 2b verläßt daher als halbverpacktes Produkt 51b die Faltvorrichtung 29b und gelangt sodann in die Vorrichtung 41b (Fig. 11) zum Verprägen der seitlichen Ränder 45b und 46b des Folienstückes 24b. Als vollständig sowie einzeln verpacktes Endprodukt 52b verläßt das Hygieneprodukt 2b die Vorrichtung 41b.

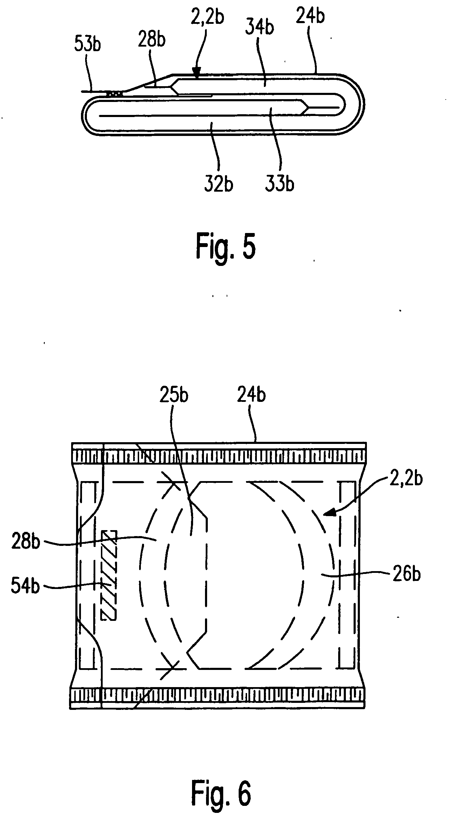

Die Anordnung der einzelnen Lagen 32b, 33b und 34b geht insbesondere auch aus Fig. 5 hervor, wobei der hintere, überstehende Rand 46b des Folienstückes 24b als Aufreißlasche 53b mit einer Klebestelle 54b auf der mittleren Lage (Fig. 5 und 6) fixiert sein kann.

Das bei der Herstellung gemäß Transportrichtung vordere Folienstükkende 25b liegt im verpackten Zustand gemäß Fig. 6 versetzt zu dem vorderen Produktende 26b. Das hintere Produktende 28b bildet gemäß Fig. 5 die obere Lage 34b im verpackten Zustand.

Grundsätzlich ist es nicht zwingend notwendig, daß die Hygieneprodukte 2, 2b in gefaltetem Zustand verpackt werden. Sie können grundsätzlich auch ungefaltet einzeln oder in Gruppen verpackt werden. Für diesen Fall ist eine Bypaßstrecke 60c im Bereich der Faltvorrichtung 29c bzw. zur Umgehung der Faltvorrichtung 29c vorgesehen, so daß ungefaltete Produkte einer Verpackungsmaschine unmittelbar zugeführt werden können.

Im Bereich der Bypaßstrecke ist das Produkt bereits ein vollkommen fertiggestelltes Produkt.

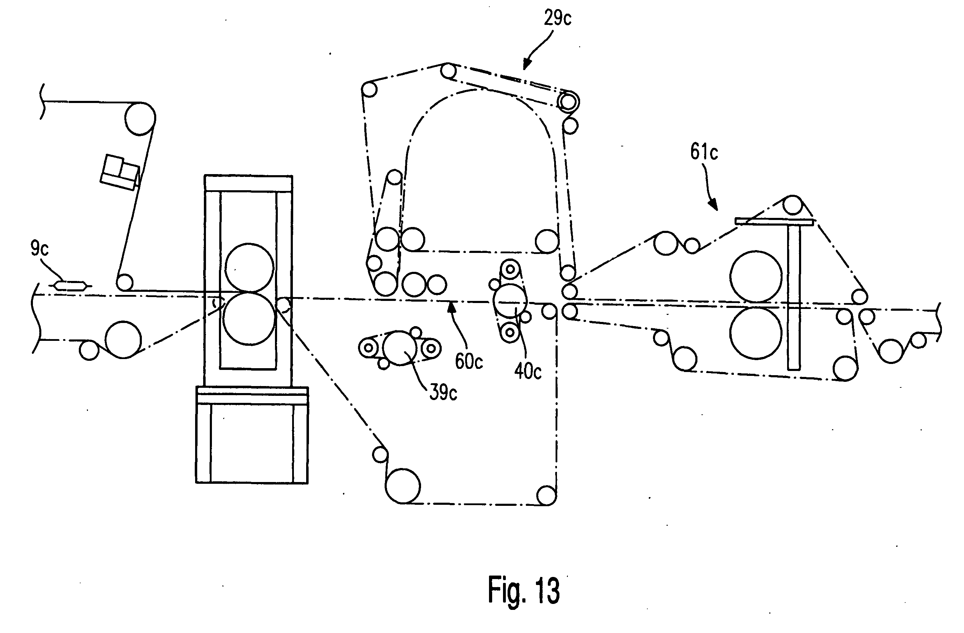

Die Bypaßstrecke 60c erstreckt sich zweckmäßigerweise in geradliniger Fortsetzung der vorgeschalteten Vorrichtung durch die Faltstation 29c. Damit die Bypaßstrecke 60c geradlinig durch die Faltstation 29c hindurchführen kann, sind die Stößeleinrichtungen 29c und 40c schwenkbar angeordnet, so daß sie im Bypaß-Betrieb aus der Bewegungsbahn der Produkte zu der Prägestation 61c hin entfernbar sind (Fig. 13).

Schließlich ist es zweckmäßig, das ein Silikonpapierstreifen an Stelle einer Folie verwendet wird, wenn die Produkte durch die Bypaßstrecke transportiert werden.

Es versteht sich schließlich, daß die Erfindung nicht auf die in den Fig. dargestellten Ausführungsbeispiele beschränkt sind, vielmehr sind auch noch Abwandlungen möglich, ohne von dem grundsätzlichen Erfindungsgedanken abzuweichen.

Wesentlich ist schließlich, daß die Produkte, nämlich absorbierende Artikel einzeln verpackt werden, wobei der mittlere Trennstreifen und Lappentrennteile dauerhaft an der Verpackung befestigt sind.

Ferner weist die Verpackung ein Klebeteil an ihrem einen Ende auf. Auch kann die Verpackung einen seitlichen Klebestreifen an einem Ende aufweisen.

Grundsätzlich sind die Längsseitenränder der Verpackung aufreißbar miteinander verbunden.

Die Faltungsachsen der Verpackung sind zu den Längsrändern der absorbierenden Einlage ausgerichtet.

Grundsätzlich geht es somit um ein Verfahren zum Verpacken einzelner, absorbierender Einlagen, die zwei Längs- und zwei Seitenränder sowie ein flüssigkeitsdurchlässiges, vorderes Flächenelement, ein flüssigkeitsundurchlässiges, hinteres Flächenelement und einen absorbierenden Kern, der zwischen den beiden Flächenelementen eingefügt ist, aufweist.

Auch geht es um die Bereitstellung einer Verpackung für ein Teil in einer allgemein flachen Lage und um das Befestigen eines mittleren Trennstreifens an der Verpackung derart, daß seine Längsachse allgemein einen rechten Winkel in Bezug auf die Längsachse der Verpakkung bildet, so wie um das Befestigen eines Paares von Lappentrennteilen an der Verpackung auf jeder Seite des mittleren Trennstreifens.

An Stelle einer Folie, einer Folienbahn oder auch eines Silikonpapierstreifens kann grundsätzlich auch jedes beliebig andere, Material verwendet werden, daß sich gleich zum Verpacken der Produkte eignet. Die Verwendung einer Folie insbesondere einer Kunststoffolie ist daher nur eine Möglichkeit zur Durchführung der Erfindung.

## Patentansprüche

1. Verfahren zum Herstellen von Hygieneprodukten (2, 2b), insbesondere von mehrlagigen, je einen Kern (3) aus saugfähigem Werkstoff und beidseitig Decklagen (4, 5) aufweisenden Damenbinden, wobei die Hygieneprodukte (2, 2b) zunächst nur als Vorprodukte (9, 9b) hergestellt und sodann mit Hilfe einer mindestens eine Silikon-Trennschicht (6, 6b) aufweisenden Folie mit Klebstoff (7, 7b) versehen werden, woraufhin die Hygieneprodukte (2, 2b) einzeln mit Hilfe derselben Folie verpackt und/oder zunächst gefaltet und durch Prägen der Folie miteinander verbunden sowie einzeln verpackt werden, **dadurch gekennzeichnet**,
a) daß zunächst Klebstoff (7, 7b) auf eine mit einer Silikon-Trennschicht (6, 6b) versehene Folie partiell in Transportrichtung bzw. in Längsrichtung der Folie aufgebracht wird,
b) daß die Folie und die Vorprodukte (9, 9b) sodann ebenfalls in Längsrichtung einander zugeführt und aufeinander gelegt werden und
c) daß die Hygieneprodukte (2, 2b) daraufhin zusammen mit der Folie gefaltet und in die Folie einzeln verpackt werden.

2. Verfahren nach Anspruch 1, **dadurch gekennzeichnet,**
**daß** die Folie in Form einer den Klebstoff (7) tragenden Folienbahn (15) auf das Vorprodukt (9) aufgelegt und erst dann durchtrennt wird.

3. Verfahren nach Anspruch 1, **dadurch gekennzeichnet,**
**daß** die Folienbahn vor dem Auflegen auf das Vorprodukt (9a, 9b) in Folienstücke (24a, 24b) geschnitten und als den Klebstoff tragendes Folienstück (24a, 24b) auf das Vorprodukt (9a, 9b) aufgelegt wird.

4. Verfahren nach mindestens einem der vorhergehenden Ansprüche,
**dadurch gekennzeichnet,**
**daß** das Folienstück (24a, 24b) in Längsrichtung versetzt auf das Vorprodukt (9a, 9b) aufgelegt wird.

5. Verfahren nach mindestens einem der vorhergehenden Ansprüche,
**dadurch gekennzeichnet,**
**daß** das Folienstück (24b) beim Abtrennen von der Folienbahn (15b) in Laufrichtung am vorderen Folienstückende (25b) und / oder am hinteren Folienstückende (27b) eine von einem geraden Schnitt abweichende Kontur erhält.

6. Verfahren nach mindestens einem der vorhergehenden Ansprüche,
**dadurch gekennzeichnet,**
**daß** das Folienstück (24b) beim Abtrennen von der Folienbahn (15b) eine Kontur derart erhält, daß der in Transportrichtung / Längsrichtung mittige Bereich (44b) des Folienstückes (24b) an mindestens einem Ende über mindestens einen der seitlichen Ränder (45b, 46b) in Laufrichtung vorsteht und / oder entgegen der Transportrichtung vorsteht.

7. Verfahren nach mindestens einem der vorhergehenden Ansprüche,
**dadurch gekennzeichnet,**
**daß** das Folienstück (24a, 24b) derart versetzt auf das Vorprodukt (9a, 9b) aufgelegt wird, daß das in Produktionsrichtung vordere Produktende (26a, 26b) mindestens teilweise das vordere Folienstückende (25a, 25b) überragt und daß das hintere Folienstückende (27a, 27b) entgegen der Transportrichtung mindestens teilweise das hintere Produktende (28a) überragt.

8. Verfahren nach mindestens einem der vorhergehenden Ansprüche,
**dadurch gekennzeichnet,**
**daß** das Vorprodukt zusammen mit dem Folienstück in einer Faltvorrichtung zu einem zwei- mehrlagigen, insbesondere zu einem dreilagigen Produkt gefaltet wird.

9. Verfahren nach mindestens einem der vorhergehenden Ansprüche,
**dadurch gekennzeichnet,**
**daß** das Hygieneprodukt (2b) nach dem Falten unter Verwendung eines überstehenden Folienstückendes (27b) und mit Hilfe von Klebestellen (54b) in dem zugleich als Verpackung dienenden Folienstück (24b) fixiert / verschlossen wird.

10. Verfahren nach mindestens einem der vorhergehenden Ansprüche,
**dadurch gekennzeichnet,**
**daß** mindestens ein Folienstückende (25b) konturiert und vorzugsweise trapezförmig kunturiert wird.

11. Verfahren nach mindestens einem der vorhergehenden Ansprüche,
**dadurch gekennzeichnet,**
**daß** das in Produktionsrichtung vordere Folienstückende (25b) trapezförmig ist.

12. Verfahren nach mindestens einem der vorhergehenden Ansprüche,
**dadurch gekennzeichnet,**
**daß** ein Folienstückende (27b) ein gerades, mittleres Randstück (47b) und nach außen jeweils anschließend bogenförmige Randstücke (48b, 49b) aufweist, die in seitliche Ränder (45b, 46b) des Folienstückes (24b) auslaufen.

13. Hygieneprodukt, insbesondere in Form einer mehrlagigen, je einen Kern (3) aus saugfähigem Werkstoff und beidseitig Decklagen (4, 5 ) aufweisenden Damenbinde, die als Vorprodukt ( 9, 9a , 9b) eine Silikon - Trennschicht ( 6, 6a) und Klebstoff ( 7, 7a, 7b ) aufweist und in einer Folie ( 8, 8b) angeordnet ist, die als Verpackung für das in Lagen ( 32, 33 bzw. 34) gelegte, einzeln verpackte Produkt vorgesehen ist, **dadurch gekennzeichnet,**
**daß** die Silikon - Trennschicht ( 6, 6a, 6b) in Längsrichtung des Produktes auf der zugleich als Verpackung dienenden Folie ( 8, 8b) angeordnet ist und
**daß** der Klebstoff ( 7, 7a, 7b ) auf der Silikon-Trennschicht ( 6, 6a, 6b) angeordnet ist.

14. Hygieneprodukt nach Anspruch 13, **dadurch gekennzeichnet,**
**daß** der Klebstoff ( 7, 7a 7b ) partiell auf der Silikon-Trennschicht ( 6, 6a, 6b ) angeordnet ist.

15. Hygieneprodukt nach Anspruch 14, **dadurch gekennzeichnet, daß** die Silikon - Trennschicht ( 6, 6a, 6b) eine durchgehend streifenförmige Fläche bildet.

16. Hygieneprodukt nach Anspruch 14, **dadurch gekennzeichnet, daß** die Silikon - Trennschicht ( 6, 6a, 6b ) in Längsrichtung des Produktes mittig und symmetrisch auf der Folie ( 8,8b ) angeordnet ist und daß der Klebstoff ( 7, 7a, 7b ) eine kleinere, rechteckige Fläche bildend auf der Silikon - Trennschicht ( 6, 6b) angeordnet ist.

17. Hygieneprodukt nach Anspruch 14 bis 16, **dadurch gekennzeichnet,**
**daß** die Silikon - Trennschicht ( 6, 6b) die Klebstoff - Fläche in Längsrichtung zugleich fensterförmig zum einen Folienstück - Ende (27b) hin versetzt umgreift.

18. Hygieneprodukt nach Anspruch 13, **dadurch gekennzeichnet,**
**daß** die Folie als Folienstück (24a, 24b) versetzt auf dem Vorprodukt (9a, 9b) angeordnet ist.

19. Hygieneprodukt nach mindestens einem der vorhergehenden Produktansprüche, **dadurch gekennzeichnet,**
**daß** mindestens ein Folienstückende (27a, 27b) über mindestens ein Produktende (28a, 28b) vorsteht.

20. Hygieneprodukt nach mindestens einem der vorhergehenden Produktansprüche, **dadurch gekennzeichnet,**
**daß** ein Produktende (26a, 26b) über das zugehörige Folienstükkende (25a, 25b) vorsteht und daß das andere Folienstückende (27a, 27b) über das andere Produktende 28a, 28b) vorsteht.

21. Hygieneprodukt nach mindestens einem der vorhergehenden Produktansprüche, **dadurch gekennzeichnet,**
**daß** mindestens ein Folienstückende eine von einer geraden abweichende Kontur aufweist.

22. Hygieneprodukt nach mindestens einem der vorhergehenden Produktansprüche, **dadurch gekennzeichnet,**
**daß** mindestens ein Folienstückende trapezförmig ist.

23. Hygieneprodukt nach mindestens einem der vorhergehenden Produktansprüche, **dadurch gekennzeichnet,**
**daß** ein Folienstückende (27b) ein gerades, mittleres Randstück (47 b) und nach außen jeweils anschließend bogenförmige Randstücke (48b, 49b) aufweist, die in seitliche Ränder (45b, 46b) des Folienstückes (24b) auslaufen.

24. Hygieneprodukt nach mindestens einem der vorhergehenden Pro duktansprüche, **dadurch gekennzeichnet,**
**daß** die seitlichen Ränder (45b, 46b) des Folienstückes (24b) zu Verpackungszwecken verprägt sind.

25. Hygieneprodukt nach mindestens einem der vorhergehenden Produktansprüche, **gekennzeichnet durch**
eine zwei- oder mehrlagige, insbesondere dreilagige Anordnung in dem als Verpackung dienenden Folienstück.

26. Verfahren nach mindestens einem der vorhergehenden Verfahrensansprüche, **dadurch gekennzeichnet,**
**daß** ein Silikonpapierstreifen im Falle einer Bypaßstrecke an Stelle einer Folie verwendet wird.

## Claims

1. Process for manufacturing hygiene products (2, 2b) and in particular multi-layer sanitary napkins each exhibiting a core (3) made of an absorbent material and cover layers (4, 5) on both sides, wherein the hygiene products (2, 2b) are first manufactured only as pre-products (9, 9b) to which are then applied adhesive (7, 7b) with the help of a film exhibiting at least one silicone parting layer (6, 6b), whereupon the hygiene products (2, 2b) are packed individually with the help of said film and/or are first folded and then joined one with another by embossing the film as well as being individually packaged,
**characterized by**
a) adhesive (7, 7b) first being applied along the film's transport direction and/or along the longitudinal direction to a portion of a film provided with a silicone parting layer (6, 6b),
b) the film and the pre-products (9, 9b) then also being brought together, also in the longitudinal direction, and laid one on another and
c) the hygiene products (2, 2b) then being folded together with the film and packed individually in the film.

2. Process in accordance with Claim 1, **characterized by**
the film being laid on the pre-product (9) in the form of a film web (15) bearing the adhesive (7) and only then cut through.

3. Process in accordance with Claim 1, **characterized by**
the film web being cut into film blanks (24a, 24b) before being laid on the pre-product (9a, 9b) and being laid on the pre-product (9a, 9b) as a film blank (24a, 24b) bearing the adhesive.

4. Process in accordance with at least one of the foregoing claims,
**characterized by**
the film blank (24a, 24b) being laid on the pre-product (9a, 9b) in such a way as to be offset in the longitudinal direction.

5. Process in accordance with at least one of the foregoing claims,
**characterized by**
the film blank (24b) when separated from the film web (15b) receiving at its leading edge (25b) and/or at its trailing edge (27b) as seen in the longitudinal direction a contour deviating from a straight-line cut.

6. Process in accordance with at least one of the foregoing claims,
**characterized by**
the film blank (24b) upon being separated from the film web (15b) receiving a contour in such a way that the center area (44b) of the film blank (24b) along the transport direction / longitudinal direction protrudes at least at one end over at least one of the lateral edges (45b, 46b) in the longitudinal direction and/or opposite to the transport direction.

7. Process in accordance with at least one of the foregoing claims,
**characterized by**
the film blank (24a, 24b) being laid on the pre-product (9a, 9b) so as to be offset in such a way that the leading end of the product (26a, 26b) in the production direction at least in part extends beyond the leading end of the film blank (25a, 25b) and
the leading end of the film blank (27a, 27b) opposite the transport direction at least in part extends beyond the trailing end of the product (28a).

8. Process in accordance with at least one of the foregoing claims,
**characterized by**
the pre-product being folded in a folding device together with the film blank to form a two- or multi-layered product and in particular a three-layered product.

9. Process in accordance with at least one of the foregoing claims,
**characterized by**
the hygiene product (2b) after folding and using a protruding end of the film blank (27b) and with the help of adhesive areas (54b) being fixed/enclosed in the film blank (24b) serving which at the same time serves as packaging.

10. Process in accordance with at least one of the foregoing claims,
**characterized by**
at least one end of the film blank (25b) being contoured and preferably trapezoidally contoured.

11. Process in accordance with at least one of the foregoing claims, **characterized by**
the leading end of the film blank (25b) in production direction being trapezoidal.

12. Process in accordance with at least one of the foregoing claims,
**characterized by**
an end of the film blank (27b) exhibiting a straight middle edge section (47b) and outwardly from that to each side arc-shaped edge areas (48b, 49b) which run out at the side edges (45b, 46b) of the film blank (24b).

13. Hygiene product in particular in the form of a multi-layer sanitary napkin exhibiting a core (3) made of an absorbent material and cover layers (4, 5) on both sides which as a pre-product (9, 9a, 9b) exhibits a silicone parting layer (6, 6a) and adhesive (7, 7a, 7b) and is located in a film (8, 8b) which is provided as packaging for the individually packaged products, folded in layers (32, 33 and/or 34), **characterized by** the silicone parting layer (6, 6a, 6b) being located in the longitudinal direction of the product on the film (8, 8b) which at the same time serves as packaging and
the adhesive (7, 7a, 7b) being located on the silicone parting layer (6, 6a, 6b).

14. Hygiene product in accordance with Claim 13, **characterized by** the adhesive (7, 7a, 7b) being located in part on the silicone parting layer (6, 6a, 6b).

15. Hygiene product in accordance with Claim 14, **characterized by** the silicone parting layer (6, 6a, 6b) forming a continuous, stripe-shaped area.

16. Hygiene product in accordance with Claim 14, **characterized by** the silicone parting layer (6, 6a, 6b) being located in the center and symmetrically on the film (8, 8b) in the longitudinal direction and by the adhesive (7, 7a, 7b) being located and forming a rectangular area on the silicone parting layer (6, 6b).

17. Hygiene product in accordance with Claim 14 to 16, **characterized by**
the silicone parting layer (6, 6b) wrapping around the adhesive surface with an offset in the longitudinal direction and like a window toward one end of the film blank (27b).

18. Hygiene product in accordance with Claim 13, **characterized by** the film as a film blank (24a, 24b) being located offset on the pre-product (9a, 9b).

19. Hygiene product in accordance with at least one of the foregoing claims, **characterized by**
at least one end of the film blank (27a, 27b) extending beyond one end of the product (28a, 28b).

20. Hygiene product in accordance with at least one of the foregoing claims, **characterized by**
a product end (26a, 26b) extending beyond the associated end of the film blank (25a, 25b) and by the other end of the film blank (27a, 27b) extending beyond the other end of the product (28a, 28b).

21. Hygiene product in accordance with at least one of the foregoing claims, **characterized by**
at least one end of the film blank exhibiting a contour deviating from a straight line.

22. Hygiene product in accordance with at least one of the foregoing claims, **characterized by**
at least one end of the film blank being trapezoidal.

23. Hygiene product in accordance with at least one of the foregoing claims, **characterized by**
an end of the film blank (27b) exhibiting a straight middle edge section (47b) and outwardly from that to each side arc-shaped edge areas (48b, 49b) which run out at the side edges (45b, 46b) of the film blank (24b).

24. Hygiene product in accordance with at least one of the foregoing claims, **characterized by**
the side edges (45b, 46b) of the film blank (24b) being embossed for packaging purposes.

25. Hygiene product in accordance with at least one of the foregoing claims, **characterized by**
a two- or multi-layer and in particular three-layer arrangement inside the film blank serving as packaging.

26. Process in accordance with at least one of the foregoing claims, **characterized by**
a strip of silicone paper being used in place of a film when the product is transported via the bypass section (60).

## Revendications

1. Procédé de fabrication de produits hygiéniques (2, 2b), en particulier de serviettes hygiéniques de plusieurs couches, lesdites serviettes comportant chacune un noyau (3) d'une matière absorbante et des couches de couverture des deux côtés (4, 5), lesdits produits hygiéniques (2, 2b) ne sont tout d'abord conçus qu'en tant qu'avant-produit (9, 9b) et sont ensuite munis d'une substance adhésive à l'aide d'une feuille comportant au moins une couche de séparation en silicone (6, 6b), les produits hygiéniques (2, 2b) étant séparément emballés à l'aide de la même feuille et / ou d'abord pliés et liés à la feuille par gaufrage et emballés séparément, **caractérisé en ce que**
a) une substance adhésive (7, 7b) est appliquée partiellement sur une feuille munie d'une couche de séparation en silicone (6, 6b) dans le sens du transport et dans le sens de la longueur de la feuille,
b) la feuille est ensuite posée sur l'avant-produit (9, 9b) dans le sens de la longueur et
c) les produits hygiéniques (2, 2b) sont alors pliés avec la feuille et emballés séparément dans celle-ci.

2. Procédé selon la revendication 1, **caractérisé en ce que**
la feuille est d'abord posée sur l'avant-produit (9) sous la forme d'une bande de feuille (15) portant la substance adhésive (7) pour être coupée seulement ensuite.

3. Procédé selon la revendication 1, **caractérisé en ce que**
la bande est coupée en morceaux de feuille (24a, 24b) avant que la feuille ne soit posée sur l'avant-produit (9a, 9b) et est posée sur l'avant-produit (9a, 9b) en tant que morceau (24a, 24b) portant la substance adhésive.

4. Procédé selon au moins l'une des revendications précédentes,
**caractérisé en ce que**
le morceau de feuille (24a, 24b) est placé de manière décalée sur l'avant-produit (9a, 9b) dans le sens de la longueur.

5. Procédé selon au moins l'une des revendications précédentes,
**caractérisé en ce que**
le morceau de feuille (24b), lorsqu'il est détaché de la bande de feuille (15b), prend dans le sens de la marche une forme déviant de la coupe droite à son extrémité avant (25b) et / ou arrière (27b).

6. Procédé selon au moins l'une des revendications précédentes,
**caractérisé en ce que**
le morceau de feuille (24b), lorsqu'il est détaché de la bande de feuille (15b), prend une forme telle que la partie centrale (44b) du morceau de feuille (24b) dans le sens du transport / de la longueur dépasse au moins sur une extrémité au moins un des bords latéraux (45b, 46b) dans le sens de la marche et / ou dans le sens opposé au transport.

7. Procédé selon au moins l'une des revendications précédentes,
**caractérisé en ce que**
le morceau de feuille (24a, 24b) est posé sur l'avant-produit (9a, 9b) de manière décalée, de sorte que l'extrémité avant du produit dans le sens de production (26a, 26b) dépasse au moins en partie l'extrémité avant du morceau de feuille (25a, 25b) et que l'extrémité arrière du morceau de feuille (27a, 27b) dépasse dans le sens opposé au transport au moins en partie l'extrémité arrière du produit (28a).

8. Procédé selon au moins l'une des revendications précédentes,
**caractérisé en ce que**
l'avant-produit est plié avec le morceau de feuille dans un dispositif de pliage pour former un produit plié en deux ou plusieurs couches, en particulier en trois couches.

9. Procédé selon au moins l'une des revendications précédentes,
**caractérisé en ce que**
le produit hygiénique (2b), après avoir été plié, est fixé / fermé, à l'aide d'une extrémité du morceau de feuille qui déborde (27b) et au moyen de points de collage (54b), dans le morceau de feuille (24b), qui fait aussi office d'emballage.

10. Procédé selon au moins l'une des revendications précédentes,
**caractérisé en ce que**
au moins une extrémité du morceau de feuille (25b) prend une forme particulière, de préférence une forme de trapèze.

11. Procédé selon au moins l'une des revendications précédentes,
**caractérisé en ce que**
l'extrémité avant du morceau de feuille (25b) dans le sens de production revêt une forme de trapèze.

12. Procédé selon au moins l'une des revendications précédentes,
**caractérisé en ce que**
une extrémité du morceau de feuille (27b) présente un bord droit central (47b) et des bords extérieurs de forme arrondie (48b, 49b), qui s'étendent dans les bords latéraux (45b, 46b) du morceau de feuille (24b).

13. Produit hygiénique, en particulier sous la forme d'une serviette hygiénique de plusieurs couches, ladite serviette comportant un noyau (3) de matière absorbante et des couches de couverture des deux côtés (4, 5), qui, en tant qu'avant-produit (9, 9a, 9b) comprend une couche de séparation en silicone (6, 6a) et une substance adhésive (7, 7a, 7b) et est disposé dans une feuille (8, 8b) qui fait office d'emballage du produit plié en plusieurs couches (32, 33 et 34) et emballé séparément,
**caractérisé en ce que**
la couche de séparation en silicone (6, 6a, 6b) est disposée dans le sens de la longueur du produit sur la feuille (8, 8b) faisant aussi office d'emballage et la substance adhésive (7, 7a, 7b) est disposée sur la couche de séparation en silicone (6, 6a, 6b).

14. Produit hygiénique, selon revendication 13,
**caractérisé en ce que**
la substance adhésive (7, 7a, 7b) est partiellement disposée sur la couche de séparation en silicone (6, 6a, 6b).

15. Produit hygiénique, selon revendication 14,
**caractérisé en ce que**
la couche de séparation en silicone (6, 6a, 6b) forme une surface rayée continue.

16. Produit hygiénique, selon revendication 14,
**caractérisé en ce que**
la couche de séparation en silicone (6, 6a, 6b) est disposée centriquement et symétriquement sur la feuille (8, 8b) dans le sens de la longueur du produit et que la substance adhésive (7, 7a, 7b) est disposée sur la couche de silicone (6, 6b) formant une surface rectangulaire de plus petite taille.

17. Produit hygiénique, selon les revendications 14 à 16,
**caractérisé en ce que**
la couche de séparation en silicone (6, 6b) entoure de manière décalée dans le sens de la longueur la surface recouverte de substance adhésive, formant ainsi une fenêtre à une extrémité du morceau de feuille (27b).

18. Produit hygiénique, selon la revendication 13,
**caractérisé en ce que**
la feuille est posée de manière décalée sur l'avant-produit en tant que morceau de feuille (24a, 24b).

19. Produit hygiénique selon au moins l'une des revendications précédentes, **caractérisé en ce que**
au moins une extrémité du morceau de feuille (27a, 27b) dépasse au moins une extrémité du produit (28a, 28b).

20. Produit hygiénique selon au moins l'une des revendications précédentes, **caractérisé en ce que**
une extrémité du produit (26a, 26b) dépasse l'extrémité du morceau de feuille correspondant (25a, 25b) et que l'autre extrémité du morceau de feuille (27a, 27b) dépasse l'autre extrémité du produit (28a, 28b).

21. Produit hygiénique selon au moins l'une des revendications précédentes, **caractérisé en ce que**
au moins une extrémité du morceau de feuille présente une forme non droite.

22. Produit hygiénique selon au moins l'une des revendications précédentes, **caractérisé en ce que**
au moins une extrémité du morceau de feuille présente une forme de trapèze.

23. Produit hygiénique selon au moins l'une des revendications précédentes, **caractérisé en ce que**
une extrémité du morceau de feuille (27b) présente un bord droit central (47b) et des bords extérieurs de forme arrondie (48b, 49b), qui s'étendent dans les bords latéraux (45b, 46b) du morceau de feuille (24b).

24. Produit hygiénique selon au moins l'une des revendications précédentes, **caractérisé en ce que**
les bords latéraux (45b, 46b) du morceau de feuille (24b) sont gaufrés à des fins d'emballage.

25. Produit hygiénique selon au moins l'une des revendications précédentes, **caractérisé par**
une disposition de deux ou plusieurs couches, en particulier de trois couches, dans le morceau de feuille faisant office d'emballage.

26. Procédé selon au moins l'une des revendications précédentes,
**caractérisé en ce que**
une bande de papier en silicone est utilisée à la place d'une feuille dans le cas d'une voie en dérivation (60).
